# EUROPEAN PATENT SPECIFICATION

(11) **EP 0 450 737 B1**
(45) Date of publication and mention of the grant of the patent: **17.01.1996**
(21) Application number: 91201378.6
(22) Date of filing: 22.11.1988
(51) Int. Cl.: A61M 1/38

(54) **Single needle continuous hemapheresis apparatus and methods**
Vorrichtung und Verfahren zur kontinuierlichen Einnadel-Hemapherese
Procédés et appareil d'hémaphérèse en continu à aiguille unique

(30) Priority: 25.11.1987 US 125102
(43) Date of publication of application: 09.10.1991
(62) Divisional of application: 89901807.1
(73) Proprietor: BAXTER INTERNATIONAL INC. (a Delaware corporation), Deerfield Illinois 60015 (US)
(72) Inventor: Schoendorfer, Donald W., Santa Ana, CA 92705 (US); Prince, Paul R., San Juan Capistrano, CA 92675 (US)
(74) Representative: MacGregor, Gordon

(56) References cited:
- EP-A- 0 131 529
- EP-A- 0 156 496
- EP-A- 0 171 749
- WO-A-86/05999
- US-A- 4 061 031
- US-A- 4 643 714

## Description

The present invention relates to single needle hemapheresis apparatus and methods and particularly relates to apparatus and methods for the continuous separation of blood constituents from whole blood in a single needle hemapheresis system.

There are a number of automated on-line donor hemapheresis systems for separating whole blood into two or more of its constituents including, for example, plasma, blood cell concentrate, and platelet-rich plasma. Such systems are designed to collect a predetermined volume of plasma from a donor using a fully-automated processing program in conjunction with a hemapheresis instrument and a disposable tubing or harness set packaged separately from the instrument. One such system is the AUTOPHERESIS-C Plasmapheresis System manufactured by Baxter Healthcare Corporation, a subsidiary of the assignee of the present invention.

Typically in systems of this general type, the instrument includes a microprocessor for controlling a number of pumps, clamps, detectors, monitoring systems, etc., for automating the collection of whole blood from the donor, separating the blood into plasma and cell concentrate, collecting the plasma and reinfusing the cell concentrate into the donor using a disposable tubing or harness set applied to the instrument. Generally, the disposable tubing set may include a venous phlebotomy needle for whole blood collection and blood concentrate reinfusion, a separator for separating anticoagulated whole blood into plasma and cell concentrate, a plasma collection container for receiving the plasma from the separator, a reinfusion reservoir from which cell concentrate flows back to the donor during reinfusion and various tube runs for connection with other parts of the instrument and its various pumps, clamps and detectors. Thus, upon application of the tubing set to the instrument and performing various setup procedures, including venepuncture on the blood donor, the instrument operates to alternate between collection and reinfusion cycles. In the collection cycle, anticoagulated whole blood is pumped by a blood pump to the separator of the tubing set where it is separated into plasma which flows to a collection container and cell concentrate which flows to the reinfusion reservoir. In the reinfusion cycle, the blood pump reverses to flow cell concentrate from the reservoir through the phlebotomy needle to the donor.

Such system is adapted for blood cell separation generally and may be adapted specifically for platelet separation. In general, such systems collect whole blood from the donor, separate off the desired cells, and return the remaining blood components to the donor through a single needle. While a return needle separate from the collection needle can also be used in systems of this general type, for example see U.S. patent application Serial No. 644,032, filed August 24, 1984, a single venepuncture needle for both collection and reinfusion offers many advantages, including patient comfort and cost minimization. In such systems, the alternate collection and return cycles should be short because it is important not to remove excessive volumes of blood from the donor at any one time.

It has been discovered that the magnitude of the driving pressures, during blood processing, to remove whole blood from the vein through one needle and return packed cells to the donor through another needle, varies with time. For example, it has been discovered that, the pressure needed to remove blood from a donor's vein starts out slightly positive and becomes negative until it suddenly returns to a less negative value. While this phenomenon was discovered in a dual-needle system, that is, a system where collection from the donor is accomplished through one needle and reinfusion through another, it is also applicable to single-needle systems. Particularly, it is believed that the phenomenon occurs because of a lack of anticoagulant in a first segment of the blood supply line of the tubing or harness set between the single phlebotomy needle (the blood collection needle in a dual-needle system or the single blood collection and reinfusion needle of a single-needle system) and the Y-junction with the anticoagulant line. For example, there is a blood line segment on the order of about twelve inches of tubing between the needle and the Y-junction with the anticoagulant line. It appears that clots form in this tubing segment. Because the clots tend to occlude the tube, the pressure created to maintain a constant flow rate increases to maintain the blood flow constant. Eventually, however, the clot breaks loose and is carried downstream by the blood flow, whereupon the pressure returns to a less negative value. Activation of the blood in this manner is believed to have detrimental effects on bath platelet quality and platelet-rich plasma yield. With regard to the latter, activation causes platelet clumping and clumped platelets respond to the centrifugal force in the separator in a manner similar to red cells. Thus, clumped platelets tend to get separated with the red cells and are returned to the donor, thereby reducing platelet yield.

It has therefore been found desirable to avoid or overcome this cause of the variation in pressure in a single-needle hemapheresis system. One way of accomplishing this, is to prime anticoagulated blood into the reservoir, while simultaneously pumping at a lower rate the contents of the reservoir through the cell separator at a lesser pump rate. Once the reservoir is full, the blood pump is reversed to pump blood from the reservoir for reinfusion into the donor. During reinfusion, however, blood also is pumped from the reservoir to the separation device whereby the separation device continuously separates the blood constituents. In this manner, interruptions in the supply of blood to the reservoir will not affect the platelet separation process. Importantly, clotting tendencies will be diminished in the un-anticoagulated portion of the tubing inasmuch as the blood flow direction in the needle changes rapidly in the course of the collection and reinfusion cycles. Consequently, by frequently reversing the flow of blood through the phlebotomy needle, clot formation is attenuated.

While the foregoing provides a practical solution, it does not take into account the potential problem of mixing unprocessed blood with processed blood. That is to say, the use of a reservoir with a single compartment which is sequentially filled and emptied by a single pump and sampled continuously by another pump for flowing blood to the separation device, necessarily mingles processed and unprocessed blood.

Consequently, and in accordance with a preferred aspect of the present invention, there is provided a single-needle continuous hemapheresis system which provides for substantial isolation of unprocessed blood from processed blood in a single reservoir. This is accomplished by substantially segregating the reservoir of the harness set into two compartments by an internal baffle.

One practical problem arises. It is important that the system which controls the filling and emptying of each reservoir side know precisely how full each one is. Thus, duplication of the number of sensors would be required. This means relatively high costs and complexity. Another approach can be to put undesirable mixing of the two sides of the reservoirs through the through passage can be minimized if the liquid levels in the two sides are kept close to each other. Consequently, a further aspect of the present invention provides for substantial equalization of the flows into and out of the compartments for both unprocessed blood and processed blood in all phases and modes of operation so that the liquid levels remain substantially equal with a minimum of flow through the lower level equalizing port between the compartments. Substantial separation of unprocessed and processed blood is therefore provided.

In accordance with a still further preferred aspect of the present invention, there is provided a tubing or harness set employing a dual-compartment reservoir, similarly as described above, but wherein the fluids in the compartments are completely isolated one from the other except for an overflow port adjacent the top of the reservoir. It must be recognized, however, that the hardware instrumentation for use with this harness set remains the same as in the previously discussed embodiment. In such instrumentation, there are provided, among pumps, clamps, detectors and the like, four sensors vertically superposed one over the other for determining the volume of blood in the reservoir. Where the previously-described system used two compartments with a communicating port at the lower portion of the baffle defining the compartments, such sensors could readily detect various liquid levels in the reservoir. However, when providing two isolated compartments, it was difficult, although highly desirable, to determine the various liquid levels in each compartment using the same instrumentation. That is, these same sensors must determine the fluid level in each of the two compartments in order to properly cycle the instrument. Consequently, the interior baffle dividing the reservoir into the two isolated compartments, in accordance with the present invention, is specifically designed to accommodate and enable use of the sensors of the extant instrument. The baffle is thus formed in a series of lateral offsets or a vertical zigzag shape such that portions of one compartment lie in vertical registry over portions of another compartment and in lateral registry with the sensors. In this manner, two of the four sensors may be used to sense the liquid level in one of the compartments, while the other two sensors may be used to sense liquid levels in the other compartment, all of the sensors lying in vertical registration one with the other.

EP-A-0171749 discloses apparatus for separating blood into components in which blood is pumped from a donor directly to a separator. A component is collected from the separator in a reservoir and reinfused to the donor. In the reinfusion step, some of the component in the reservoir is fed back to the separator, so that there is continuous operation.

In another embodiment, blood is fed from a donor to a reservoir and from the reservoir to the separator. The separated component is also fed to the same reservoir and, during reinfusion, a mixture of whole blood and the separated component is fed to the separator for continuous separation.

The precharacterising part of Claim 1 is based on this disclosure and the distinguishing features of the apparatus of the invention are set out in the characterising part of Claim 1. The invention also includes a method and harness set as claimed in Claims 9 and 15 respectively.

Thus, it is a primary object of the present invention to provide novel and improved hemapheresis apparatus and methods for continuously separating blood constituents from whole blood while alternating blood collection and reinfusion cycles, preferably in a single-needle system, and precluding clot formation in un-anticoagulated portions of the whole blood conduit.

These and further objects and advantages of the present invention will become more apparent upon reference to the following specification, appended claims and drawings.

### DETAIL DESCRIPTION OF THE DRAWING FIGURES

Figure 1 is a perspective view of a hemapheresis instrument with a harness set constructed in accordance with the present invention and applied to the instrument;
Figure 2 is a front elevational view of another form of instrument and harness set applied thereto constructed in accordance with the present invention;
Figures 3 and 4 are side and front cross-sectional views of the reservoir used in the harness set applied to the instrument of Figure 2;
Figure 5 is a schematic view of portions of the harness set applied to the instrument of Figure 2 with charts illustrating the flow rates at various stages of the process and at different locations in the harness set;
Figure 6 is a still further embodiment of an instrument and harness set applied thereto constructed in accordance with the present invention;
Figure 7 is a perspective view with parts broken out and in cross-section of the reservoir used in the harness set illustrated in Figure 6; and
Figure 8 is an enlarged fragmentary longitudinal cross-sectional view through the reservoir illustrated in Figure 7.

### DETAILED DESCRIPTION OF A PREFERRED EMBODIMENT

Reference will now be made in detail to the present preferred embodiment of the invention, an example of which is illustrated in the accompanying drawings.

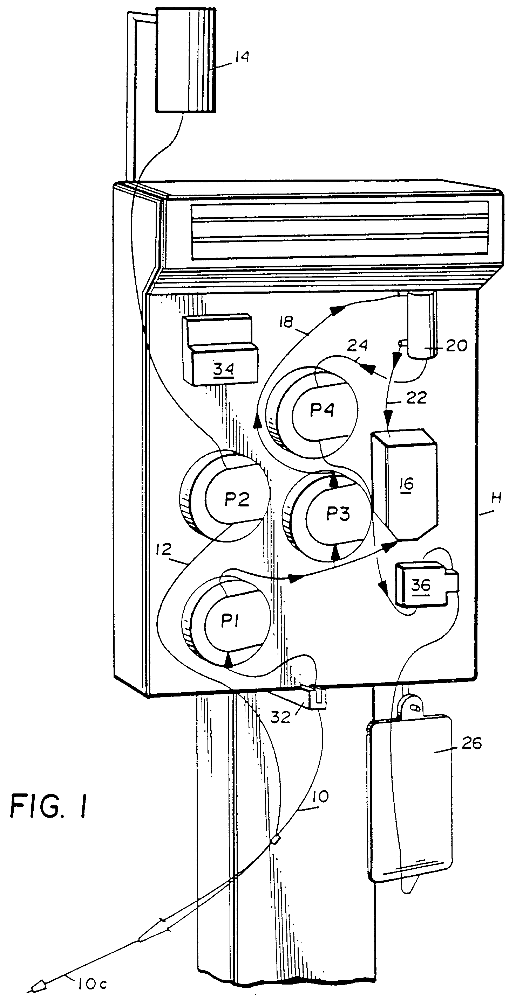

Referring now to Figure 1, there is illustrated a hemapheresis system for use in practicing one aspect of the present invention. In this system, there is provided a tubing or harness set for application to the hemapheresis instrument H. The harness set includes a venepuncture needle set, not shown, comprised of a single needle for receiving whole blood from a donor and reinfusing a separated second component into the donor. The venepuncture needle set communicates with a blood line 10. An anticoagulant line 12 joins blood line 10 adjacent the venepuncture needle set through a Y-connection. Anticoagulant line 12 communicates with an anticoagulant supply 14 through suitable connections. It will be appreciated that there is a distance of blood line tubing 10 (10C) between the needle set and the Y-connection with the anticoagulant line 12 in which whole blood flows without anticoagulant. It is this length of tubing in which the clots may form, as previously discussed.

The tubing or harness set also includes a reservoir 16 and a branch conduit 18 which communicates anticoagulated whole blood in blood line 10 into a blood component separator 20, also forming part of the harness. A conduit 22 communicates between separator 20 and reservoir 16 for directing the separated second component into reservoir 16. A conduit 24 communicates between the lower end of separator 20 and a separated primary component collection bag 26. It will be appreciated that the aforedescribed tubing set or harness is manufactured and sold as a disposable for application to a hemapheresis instrument which, when operated in conjunction with the applied tubing set, performs the collection, reinfusion and separating functions.

The hemapheresis system hereof comprises both the harness set and the instrument. Generally, the system, with the harness set applied, requires a single venepuncture and alternates between collection of whole blood and reinfusion of residual concentrate while continuously separating whole blood into constituents using a system of pumps, clamps and sensors controlled by a microprocessor. Particularly, instrument H includes a plurality of pumps to which the harness set is applied to pump the various fluids. For example, blood line or tubing 10 is applied to a pump P1 for pumping blood to and from the donor to reservoir 16. The anticoagulant line 12 is applied to pump P2 for pumping anticoagulant from supply container 14 through anticoagulant line 12 into blood line 10 adjacent the venepuncture needle. Tubing 18 is applied to a pump P3 connecting between blood line 10 and an inlet port to separator 20. Tubing 24 is applied to pump P4 for pumping the desired separated component from separator 20 to the collection bag 26. Pumps P1, P2, P3 and P4 are preferably of a peristaltic type. It will be appreciated that there are various other detection devices and other features provided on the face of the instrument, for example, an air detector 32 for detecting air in the blood line, a pressure transducer assembly 34, which is coupled to the harness, by means not shown, a hemoglobin detector 36 and other sensors and clamps, all of which need not be described herein for an understanding of the present invention.

The operation of instrument H is under the control of a microprocessor. In use, the harness set is applied to the instrument and various checks are made by the instrument and the phlebotomist to ensure that the instrument is functioning properly. Venepuncture is then performed and pump P2 is actuated. Pump P2 pumps anticoagulant from supply 14 through line 12 into the blood line immediately behind the venepuncture needle. Pump P1 is also activated to pump anticoagulated blood through line 10 into reservoir 16 to prime the latter. At the same time, pump P3 is actuated to pump blood from blood line 10 into separator 20. It will be appreciated that, according to the present invention, the volumetric flow pumped by pump P3 through line 18 to separator 20 is less than the volumetric flow of anticoagulated blood in line 10. Therefore, reservoir 16 will eventually fill with anticoagulated blood. Pump P4 is also actuated to pump the desired blood component product separated from anticoagulated whole blood by separator 20 for delivery through line 24 to collection bag 26. Consequently, it will be appreciated that blood is collected from the donor and simultaneously separated by separator 20. The separated blood product from separator 20 flows continuously into reservoir 16 via line 22. Thus, during this collection and separation stage, reservoir 16 is being filled by the portion of the donor's blood not pumped into separator 20 through line 18 as well as by the residual blood components, i.e., red cells and white cells, exiting separator 20 via line 22.

Once the reservoir has filled, an optical sensor, not shown, on the instrument, senses the level of blood in the reservoir and, in response thereto, the microprocessor reverses the direction of blood pump P1. This initiates the reinfusion cycle, whereby blood is pumped from reservoir 16 through blood line 10 by pump P1 for reinfusion into the donor through the single venepuncture needle. Simultaneously and continuously therewith, blood is pumped from line 10 by pump P3 for delivery through line 18 to separator 20. At the same time, residual blood flows from separator 20 into reservoir 16. Consequently, reservoir 16 is drained of blood by pump P1 during reinfusion and by pump P3 which delivers blood to separator 20. Simultaneously, residual blood flows into reservoir 16 via line 22. Consequently, reservoir 16 empties. When the reservoir is close to being completely emptied, a sensor, not shown, senses the level of fluid in the reservoir and, in response, the microprocessor once again reverses pump P1, whereupon the instrument repeats the previously described collection cycle. Thus, during both collection and reinfusion stages, separator 20 operates continuously to effect separation.

By operating the system of the present invention in this manner, the collection/reinfusion cycles are alternated relatively rapidly, i.e., within a few minute's time. By effecting rapid alternate collection and reinfusion cycles, the un-anticoagulated portion of tubing 10 between the venepuncture needle and the Y-connection with the anticoagulant line 12 is rapidly back-flushed with anticoagulated blood and in sufficient time to preclude clot formation in that portion of the blood line. Consequently, the abrupt pressure changes discovered, as previously mentioned, are eliminated and the potentially adverse effects of such pressure changes on the blood including clotting are likewise eliminated.

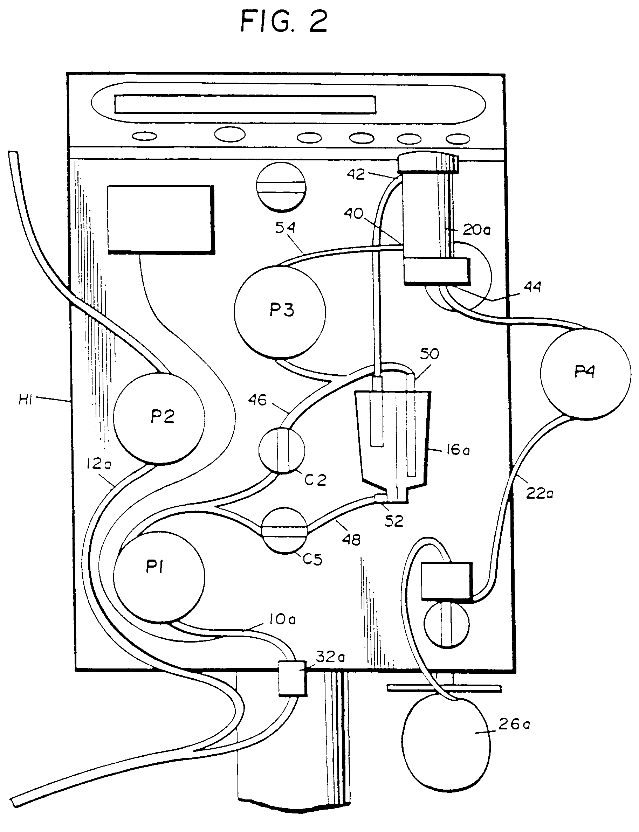

Turning now to the embodiment of the present invention illustrated in Figures 2-5, there is illustrated apparatus for substantially isolating unprocessed and processed blood in a single reservoir. This is accomplished by providing an internal baffle in the reservoir to divide it into two discrete compartments except for a bypass port at the bottom of the baffle and by control of input processed and unprocessed blood to the reservoir whereby liquid levels in the compartments of the reservoir are equalized and substantial isolation between unprocessed blood and processed blood is maintained. In the previous embodiment, it will be appreciated that the processed and unprocessed blood were mixed in a single reservoir. It has been found desirable, however, to maintain such processed and unprocessed bloods substantially segregated. However, the existing hemapheresis instrument imposes certain constraints or the design of a system for maintaining processed and unprocessed bloods segregated in a single reservoir. For example, it is necessary to determine the volume of blood in the two compartments of the reservoir which store processed and unprocessed blood, respectively. Therefore, while it is considered desirable to maintain the processed and unprocessed blood segregated, any such system for segregating the blood has to be compatible with the instrument and particularly with the sensors extant on the instrument for determining liquid level in the previously single compartmented reservoir. According to this embodiment of the invention, two substantially separate compartments are provided in the reservoir and constant liquid levels are maintained by providing for control of the flow into and out of the two compartments, thereby eliminating or minimizing significant flow from one compartment to the other through the bypass port, and enabling blood level sensing to be accomplished by sensors provided on an existing instrument.

Accordingly, in Figure 2, there is illustrated an instrument H1 substantially similar to the instrument H described previously. Here, motor P4 is disposed to one side of the instrument rather than on its face. Also, clamps C2 and C5 are provided. The tubing or harness set is also somewhat differently arranged and includes a platelet separator 20a, for example, as described and illustrated in U.S. patent application Serial No. 644,032. That separator, for example, has three ports, a blood inlet port 40, packed blood cell port 42 and a platelet-rich plasma port 44. Additionally, blood line 10a has a pair of branch lines 46 and 48 which extend through clamps C2 and C5, respectively, when the harness set is applied to the instrument. Line 46 extends for connection to a draw tube 48 disposed in a reservoir 16a, to be described. Line 48 connects with an inlet port 52 at the lower end of reservoir 16a. Additionally, a line 54 communicates with line 46 between draw tube 50 and clamp C2 and is connected at its opposite end to inlet port 40 of separator 20a.

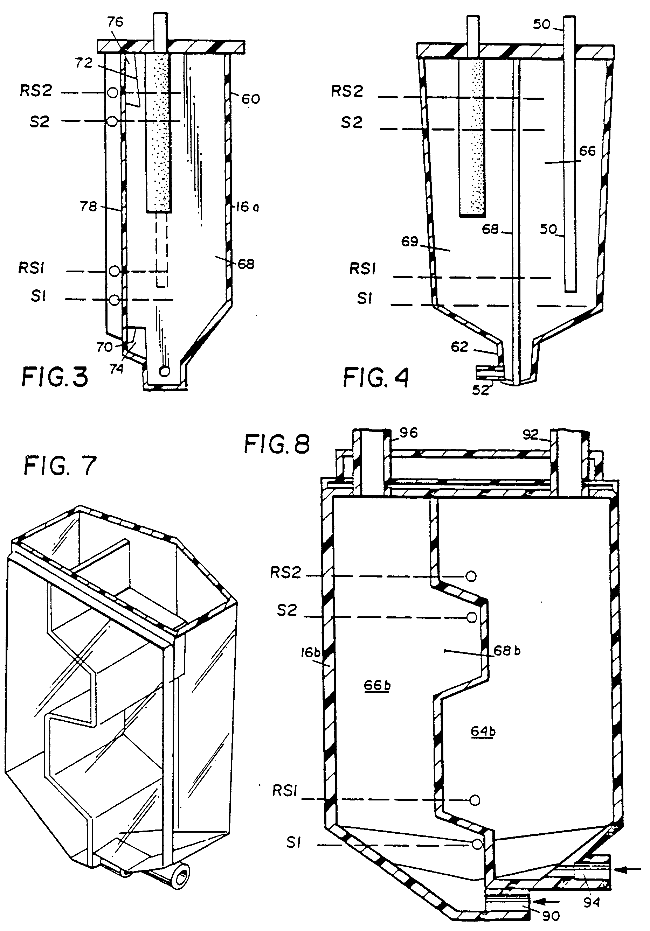

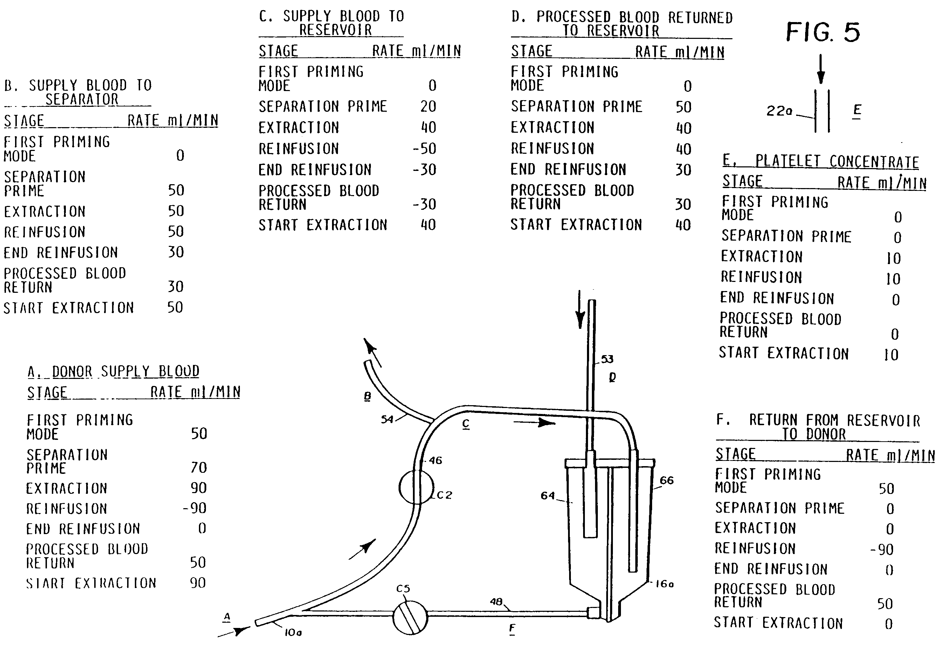

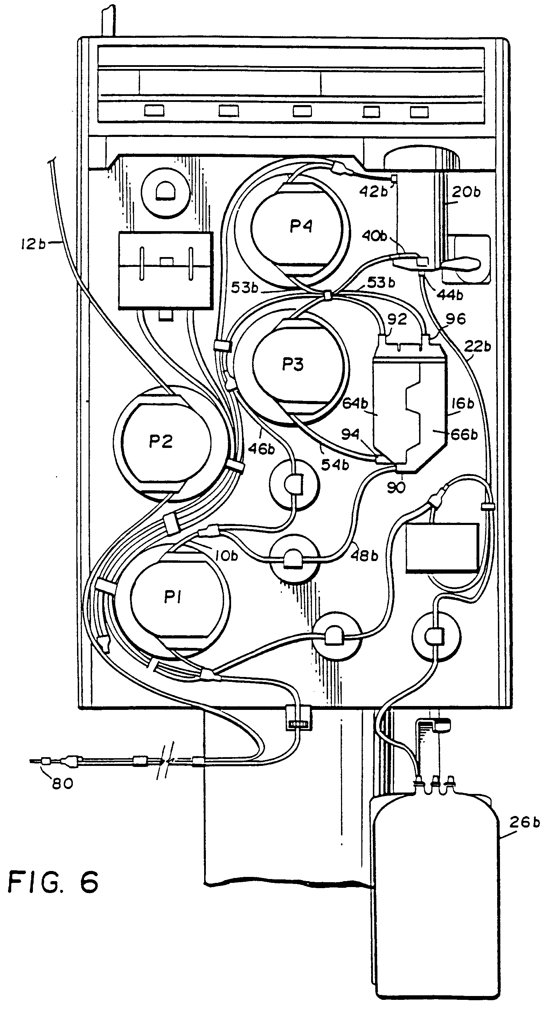

Referring now to Figures 3 and 4, the reservoir 16a of the harness set illustrated in Figure 2 will now be described. Reservoir 16a comprises a housing 60 having side, front and back walls, a top wall and a tapered lower wall terminating in a downwardly extending central channel 62. Reservoir 16a is divided into separate, side-by-side compartments 64 and 66 by a baffle 68, which extends between the top wall and into channel 62. From a review of Figures 3 and 4, it will be appreciated that baffle 68 substantially isolates compartments 64 and 66 one from the other, except for a lower cutout 70 along the lower rear side of baffle 68 and a cutout 72 along the upper rear side of baffle 68. Cutout 70 forms with the side and bottom walls of the reservoir 16a a bypass port 74 providing communication between chambers 64 and 66. Cutout 72 at the upper end of the baffle provides, in conjunction with the back wall of reservoir 16a, an overflow passage 76. The rear wall of the reservoir has a rearwardly extending channel 78, which is received in a portion of the instrument face for purposes of measuring fluid levels. Card readers are provided on the front panel of the instrument and their locations are designated by the identifying characters S1, RS1, S2 and RS2 and the levels thereof which are sensed are indicated by the dashed lines in Figures 4.

Referring back to Figure 2, the flow paths of the blood through the harness set when applied to the instrument H1 will now be described with respect to the various stages of operation. Once the harness has been applied to instrument H1, and all checks have been performed by the instrument and operator, the instrument enters a first priming mode. In this mode, anticoagulant pump P2 is activated to provide anticoagulant through anticoagulant line 12a into blood line 10a. Pump P1 is activated, clamp C2 is closed and clamp C5 is opened, whereby anticoagulated blood is provided through blood line 10a into the lower portion of reservoir 16a up to a level S1. In this initial prime, the blood communicates through bypass port 74 with both chambers 64 and 66, whereby the level in both chambers is substantially constant. After the reservoir prime, the separation device is primed. To accomplish this, clamp C5 is closed and clamp C2 is opened and all pumps P1-P4 are activated. Anticoagulated blood therefore flows through blood line 10a for delivery into reservoir 16a, particularly compartment 66 through draw tube 50. A portion of the anticoagulated blood delivered through line 10a is pumped by pump P3 through line 54 for delivery to separation device 20a through inlet port 40. Platelet-rich plasma is delivered from separator 20a through exit part 44 and pumped by pump P4 into collection bag 26a. Packed cells are delivered via line 53 into reservoir 16a, particularly compartment 64. Consequently, the filter of separating device 20a is primed while anticoagulated, unprocessed whole blood is delivered compartment 66 via line 46 and processed blood is delivered compartment 64 via line 53. Once the filter is primed, the system, under the control of the microprocessor, commences its blood collection cycle.

In this cycle, anticoagulated blood is delivered via blood line 10a to compartment 66 of reservoir 16a via line 46 and also separator 20a via line 54 and inlet port 40. It will be appreciated that the harness may be constructed such that the whole blood from the donor may be supplied in its entirety to the reservoir and then from the reservoir to the separator rather than being supplied in part directly to the separator from the donor and in the remaining part directly to the reservoir compartment from the donor. Packed cells flow via line 53 from separator 20a into compartment 64 of reservoir 16a while platelet-rich plasma flows into collection bag 26a via line 22a. In this collection cycle, the pump rates are set such that substantially equal flows are provided the two compartments 64 and 66 of reservoir 16a via the packed cells return line 53 and blood supply line 46. It should be noted that the blood pump rate, i.e., pump P1, is set to the sum of the supply pump rate, i.e., pump P3, and the processed flow rate through line 53. That is, the blood pump rate is equal to the supply pump rate x 2 minus the concentrate pump rate. Vein conditions causing regulation of the blood pump can be accommodated by either slowing all pumps or sacrificing some mixing of unprocessed and processed blood in the reservoir or a combination of the two. It is advantageous to make the lower level equalizing port 74 quite small so that any mixing is delayed for variations such as vein regulation or variations in concentrate flow which will occur during red-spill occurrences.

The reservoir eventually becomes full due to the influx of both processed blood and unprocessed blood through lines 53 and 46, respectively. At this stage, a reinfusion cycle is initiated. That is, the blood levels in both compartments 64 and 66 have reached the level S2 indicated in Figure 4 and the instrument sensor detects such levels. The microprocessor responds by controlling the instrument to initiate a reinfusion cycle.

To start the reinfusion cycle, the microprocessor controlled instrument causes clamp C2 to close and clamp C5 to open. Blood pump P1 is reversed and blood is drawn from the bottom of reservoir 16a for delivery via branch line 48 and blood line 10a to the single venepuncture needle for reinfusion into the donor. Additionally, blood pump P3 is maintained activated to pump blood from compartment 66 via line 54 to separator 20a. Platelet-rich plasma pump P4 is also maintained activated to flow platelet concentrate from separator 20a via line 22a into collection bag 26a. The outflow of blood from reservoir 16a via the blood lines 48 and 10a is taken substantially from compartment 64, which is being continuously supplied with processed blood via line 53. Simultaneously, blood is being removed from compartment 66 via line 54 for delivery to the separator 20a. The blood pump rates are adjusted such that the outflow from compartment 64 via blood line 48 and 10a, less the input to compartment 64 via line 53, is equal to the output via line 54 from blood compartment 66. In this manner, the levels of the processed and unprocessed blood in compartments 64 and 66, respectively, are maintained substantially equal as the overall level of the liquid in reservoir 16a decreases. It is important to note that this equalization of the flow does not cause substantial flow through bypass port 74 and therefore the processed and unprocessed bloods in the compartments 64 and 66 will not be substantially intermingled.

When the blood level reaches the level indicated RS1 in Figure 3, that low level is sensed and the microprocessor responds by indicating an end reinfusion stage. At this stage, it is necessary to stop blood pump P1 quickly, pressurize the cuff on the donor, obtain a vein control zero flow point, reverse blood pump P1 and return processed blood in the tubing into the reservoir before supply pump P3 draws air through the bottom of draw tube 50. To accomplish this, supply pump P3 is slowed along with pump P4 to reduce or stop the flow of platelet-rich plasma. The separator may also be slowed at this time.

Once the pressure cuff has been inflated and the blood pump is reversed, processed blood is returned to the reservoir from the tube set. To accomplish this, the blood pump P1 is reversed to flow blood via lines 10a and 48 into the lower part of the reservoir 16a. The supply flow through line 54 is held at a low rate and platelets are not collected. At the end of this process, the collection cycle begins and the pumps and clamps are operated as previously discussed.

A chart of representative exemplary flow rates at various locations in the system is set forth below in connection with Figure 5. The flows are given in terms of ml/min. The lefthand column of the chart below identifies the stage of the process, while the remaining column headings identify the various locations in the system. Thus, the blood flow rate at each relevant location is given for each stage of the process. In Figure 5, this information is denoted specifically by charts A-F. The headings of these charts identify the location in the system of the flow rates by corresponding letter reference A-F as applicable in drawing Figure 5. The negative signs indicate the reverse flow from that indicated by the column heading for that location and the corresponding stage.

| REPRESENTATIVE SYSTEM FLOW RATES FOR EACH RELEVANT LOCATION AND SYSTEM STAGE | | | | | | |
|---|---|---|---|---|---|---|
| | A From Donor | B Supply to Separator | C To Blood Res. | D Proc. Blood Into Res. | E Donor to Res. | F Platelet-Rich Plasma Flow |
| 1st Priming Mode | 50 | 0 | 0 | 0 | +50 | 0 |
| Separation Prime | 70 | 50 | 20 | 50 | 0 | 0 |
| Extraction (Res. Being filled) | 90 | 50 | 40 | 40 | 0 | 10 |
| Reinfusion | -90 | 50 | -50 | 40 | -90 | 10 |
| End Reinfusion | 0 | 30 | -30 | 30 | 0 | 0 |
| Process Blood Return Into Reservoir | +50 | 30 | -30 | 30 | +50 | 0 |
| Start Extraction Cycle | 90 | 50 | 40 | 40 | 0 | 10 |

Thus, for example, during the extraction cycle, whole blood flows at a rate of 90 ml/min. through blood line 10a; 50 ml/min. flow through line 54 to separator 20a; 40 ml/min. flow through line 46 into reservoir compartment 66; 40 ml/min. flow from separator port 42 of separator 20a into compartment 64 of reservoir 16a; 10 ml/min. flow from outlet port 44 of separator 20a via line 22a into collection bag 26a and 0 ml/min. flow from reservoir 16a back to the donor.

As will be appreciated, both compartments 64 and 66 empty at substantially the same rate, as well as fill at substantially the same rate. However, pump flow rate errors caused by tubing diameter, tension and input pressure to the blood pump and the supply pump will cause some mixing of processed and unprocessed blood in the first cycle. During that cycle, the known volume from RS1 to RS2, the measured pump motions, and the measured difference observed by the measure of flow through the concentrate pump enables relative calibration for later cycles. Additionally, in the first cycle, the unprocessed blood could intentionally be increased over the processed blood into the reservoir, if necessary, to bias the errors in one direction. Controlled tubing within the pumps and possibly tube stops added to the supply pump P3 would further decrease any errors in that system.

Turning now to the embodiment hereof illustrated in Figures 6-8, there is illustrated a system wherein the compartments of the reservoir are completely isolated one from the other, except for an overfill passage at the top of the reservoir. Moreover, this isolation is accomplished within the constraints of the locations of the liquid level sensors in the existing instrument. This necessitates a slightly different harness set for installation on the instrument as will now be described.

The harness set for use with this embodiment of the invention includes a blood line 10b connected with a venepuncture needle designated 80. Blood line 10b branches into lines 48b and 46b. Branch line 48b is coupled to blood line 10b through a Y-connection and to a lower outlet port 90 of reservoir 16b. Branch line 46b connects with the Y-connection with blood line 10b and an inlet port 92 for reservoir 16b. A line 54b connects between an outlet port 94 of reservoir 16b and an inlet port 40b of separator 20b. A line 53b connects between the inlet port 96 of reservoir 16b and the outlet port 42b of separator 20b. A line 22b connects between the outlet port 44b of separator 20b and with a collection bag 26b.

Referring now to Figures 7 and 8, the reservoir 16b is segregated into two discrete compartments 64b and 66b with a baffle 68b separating the compartments one from the other. The baffle forms an integral portion of the housing for the reservoir and provides for complete isolation of compartments 66b and 68b one from the other. Thus, whole blood compartment 64b has inlet port 92 and outlet port 94, while packed cell compartment 66b has inlet port 96 and outlet port 90 at its bottom. Draw tubes, now shown, are provided in communication with each of inlet ports 92 and 96, respectively, and extend into the respective compartments.

It is a particular feature of the present invention that the dual-compartment reservoir is specifically adapted for use with the sensors on the instrument face. As will be recalled, liquid levels in the reservoir are detected and signals generated thereby control the operation of the system. The reservoir illustrated in Figures 7 and 8 is specifically adapted to use those very same sensors but in a manner to effectively sense the liquid level in each of compartments 64b and 66b. To adapt the reservoir to the existing locations of the sensors, i.e., in vertical alignment one over the other, the baffle 68b has a zigzag or laterally offset cross-sections. That is to say, the baffle is comprised of laterally offset vertical portions interconnected by laterally extending portions. In this way, when the reservoir is applied to the instrument, the sensors RS2 and RS1 may be used to sense the liquid levels in compartment 64b, while the sensors S1 and S2 may be used to sense the liquid level in compartment 66b. Note that the position of the sensors is not changed.

The harness set described above is applied to the instrument by inserting the reservoir 16b in a mount, not shown, applying line 10b to pump P1, lines 46b and 48b to clamps C2 and C5, respectively, line 54b through pump P3 and line 53b through pump P4. As in previous embodiments, the anticoagulant line 12b is applied to pump P2.

In using this system, and after the donor has been connected to the phlebotomy needle 80, system checks have been made and the reservoir and separator are primed, anticoagulated blood is pumped by pump P1 through line 10a and branch line 46b past opened clamp C2 into reservoir compartment 64b via inlet port 92. Anticoagulated blood is pumped by pump P3 through line 54b and inlet port 40b into separator 20b. Packed cells from separator 20b are pumped by pump P4 through outlet 42b and via line 53b into reservoir compartment 66b. Platelet-rich plasma is delivered from outlet 44b of separator 20b through line 22b to collection bag 26b. It will be appreciated that during this collection cycle, only a portion of the whole blood volume delivered to compartment 64b is delivered to separator 20b. Simultaneously, only a portion of the whole blood volume delivered to the separator is returned to the reservoir in compartment 66b as the remaining portion is collected in bag 26b. Consequently, the liquid levels in both compartments will rise, while maintaining complete separation of the liquid input into the compartments.

When the liquid levels reach predetermined levels and are sensed, the microprocessor responds by starting the reinfusion cycle. Consequently, clamp C2 is closed and clamp C5 is opened. Pump P1 is reversed and packed cells are pumped from compartment 66b through branch line 48b, past open clamp C5 and through line 10a for reinfusion into the donor through venepuncture needle 80. Pump P3, however, continues to deliver whole blood from compartment 64b via line 54b to the separator 20b. Thus, during both collection and reinfusion cycles, separator 20b operates continuously to maintain an output of platelet-rich plasma. When the liquid levels in the reservoir compartments are again sensed prior to complete depletion of the liquids in the compartments, the microprocessor responds to initiate the collection cycle. Thus, clamp C5 is closed and clamp C2 is opened. Pump P1 is again reversed to provide anticoagulated blood into whole blood compartment 64b via lines 10b and 46b. Packed cells from separator 20b are pumped by pump P4 into the compartment 66b via line 53b. Consequently, both compartments are being filled with the respective blood fluids.

Advantageously, the packed cells and whole blood are maintained separated continuously while the cycle times remain short, precluding the clotting problem previously noted with respect to the first un-anticoagulated tube portion between the venepuncture needle and the Y-connection with the anticoagulant line. Additionally, the discontinuous collection and reinfusion cycles enable use of a single venepuncture needle and its attendant advantages.

## Claims

1. Apparatus suitable for separating blood received from a donor into constituents, comprising access means (10c) for supplying blood from a donor to a separator (20,20a) through a flow circuit (46,54,50,48) for separating the blood into first and second constituents, a reservoir (16,16a) in communication within said flow circuit with the separator (20,20a) through a first flow path (50,54), and with the access means (10c) through a second flow path (46,50), the flow circuit including a return path (48) between the separator (20,20a) and the access means (10c), operating means (P1,P3,P4) having a collection mode and a reinfusion mode, the operating means being operable in the collection mode to deliver blood from the access means (10c) to the reservoir (16,16a) and the separator (20,20a) and operable in the reinfusion mode to deliver the first constituent, separated by the separator (20,20a), to the access means (10c) through the return path (48), and means for alternating operation in said modes, the operating means (P1,P3,P4) being operable in the reinfusion mode to deliver blood from the reservoir (16,16a) to the separator (20,20a) at the same time as said delivery of the first constituent to the access means (10c), whereby there is a continuous supply of blood to the separator (20,20a), CHARACTERISED in that the flow circuit comprises a third flow path (46,54), bypassing the reservoir, between the access means (10c) and the separator (20,20a), the operating means being operable in the collection mode to deliver a quantity of blood from the access means (10c) through the third flow path (46,54) to the reservoir (16,16a) at the same time another quantity of blood is delivered from the access means (10c) to the separator (20,20a) through the first path (54).

2. Apparatus according to Claim 1 including means (16,16a) for collecting the first constituent for return to the access means (10c).

3. Apparatus according to Claim 2 wherein the collection means (16,16a) for the first constituent is the reservoir (16,16a) for collection of whole blood.

4. Apparatus according to Claim 2 wherein the collection means (64) for the first constituent is separate from the collection means (66) for whole blood.

5. Apparatus according to Claim 1 wherein, in the second mode of operation, whole blood is delivered to the separator (20,20a) substantially free of mixing with the first constituent.

6. Apparatus according to Claim 1 including conduit means between the separator (20,20a) and the reservoir (16,16a) for conveying the first constituent from the separator to the reservoir for return to the donor.

7. Apparatus according to Claim 4 including conduit means between the separator (20,20a) and the collection means (64) for the first constituent and conduit means (48) between this collection means (64) and the access means (10c).

8. Apparatus according to Claim 4 or 7 wherein the collection means for whole blood and the first constituent comprise first and second compartments (66,64) formed within a common housing (16a).

9. A method for separating blood into constituents using a separator (20,20a) for separating the blood into first and second constituents, the method comprising separately collecting the first and second constituents while supplying the blood to a reservoir (16,16a,66) and from the reservoir to the separator, and subsequently returning the first constituent, while carrying out the separation step, the method including supplying collected blood from the reservoir to the separator (20,20a) for separation while the first constituent is being returned, so that the separation step is continuous, CHARACTERISED By diverting a portion of the blood during collection to the separator, by passing the reservoir.

10. A method according to Claim 9, wherein the blood is supplied to an inlet (50) located generally at the upper portion of the reservoir (66), the reservoir having a first and second compartments (66,69), and wherein the first component is supplied from the separator (20,20a) to an inlet located generally at the upper portion of the second compartment (69) of the reservoir (16), communication being provided between the compartments (66,69).

11. A method according to Claim 10 including, during the collection cycle, supplying blood to the separator (20,20a) at a rate substantially equal to the rate at which whole blood is supplied less the rates at which the first blood component is supplied to the first compartment (66).

12. A method according to Claim 10 or 11 including, during the collection cycle, the steps of supplying blood to the first compartment (66) at a rate substantially equal to the rate at which the first blood component is supplied to the second compartment (69) whereby the blood levels in said compartments remain at substantially like levels.

13. A method according to any one of Claims 10 to 12, wherein the first and second compartments (66,69) are of a size to maintain substantially the same levels therein during both collection and returning cycles.

14. A method according to any one of Claims 10 to 13 including, during the returning cycle, the steps of supplying blood from said first compartment (66) to said separator (20,20a) at a rate substantially equal to the rate the first blood component is returned from said second compartment (69) less the rate the first blood component is supplied to said second compartment (69) from said separator (20,20a).

15. A harness set for use in apparatus for separating blood received from a donor into constituents, the harness set comprising a separator (20,20a) for separating the blood constituents, a blood reservoir (16,16a); a vein puncture needle (10c); first means (10,10a,46) for establishing blood fluid communication between said needle (10c) and the reservoir (16,16a); second means (10,24:10a,54) for establishing blood fluid communication between the needle (10c) and the separator (20,20a); third means (22,42) for establishing blood fluid communication between the separator (20,20a) and the reservoir; and fourth means (10,10a,48) establishing blood fluid communication between the reservoir (16,16a) and the needle (10c), whereby blood fluid from the donor may be collected in the reservoir (16,16a) while blood fluid is simultaneously supplied from the donor to the separator (20,20a) and whereby blood fluid from the reservoir (16,16a) may be supplied to the separator (20,20a) while blood fluid is being returned from the reservoir to the donor.

## Patentansprüche

1. Vorrichtung, die geeignet ist, um von einem Spender erhaltenes Blut in Bestandteile zu trennen, und die folgendes aufweist: eine Zugangseinrichtung (10c), um Blut von einem Spender durch einen Kreislauf (46, 54, 50, 48) einer Trenneinrichtung (20, 20a) zuzuführen, um das Blut in einen ersten und einen zweiten Bestandteil zu trennen, ein Reservoir (16, 16a), das in dem Kreislauf mit der Trenneinrichtung (20, 20a) durch eine erste Durchflußbahn (50, 54) und mit der Zugangseinrichtung (10c) durch eine zweite Durchflußbahn (46, 50) in Kommunikation ist, wobei der Kreislauf aufweist: eine Rücklaufbahn (48) zwischen der Trenneinrichtung (20, 20a) und der Zugangseinrichtung (10c), Betätigungseinrichtungen (P1, P3, P4), die eine Sammelbetriebsart und eine Reinfusionsbetriebsart haben, wobei die Betätigungseinrichtungen in der Sammelbetriebsart betätigbar sind, um Blut von der Zugangseinrichtung (10c) zu dem Reservoir (16, 16a) und zu der Trenneinrichtung (20, 20a) zu fördern, und in der Reinfusionsbetriebsart betätigbar sind, um den ersten von der Trenneinrichtung (20, 20a) getrennten Bestandteil durch die Rücklaufbahn (48) zu der Zugangseinrichtung (10c) zu fördern, und eine Einrichtung zum alternierenden Betrieb in diesen Betriebsarten, wobei die Betätigungseinrichtungen (P1, P3, P4) in der Reinfusionsbetriebsart betätigbar sind, um Blut von dem Reservoir (16, 16a) zu der Trenneinrichtung (20, 20a) gleichzeitig mit dem Fördern des ersten Bestandteils zu der Zugangseinrichtung (10c) zu fördern, so daß eine kontinuierliche Blutzuführung zu der Trenneinrichtung (20, 20a) stattfindet, DADURCH gekennzeichnet, daß der Kreislauf eine dritte Durchflußbahn (46, 54), die das Reservoir umgeht, zwischen der Zugangseinrichtung (10c) und der Trenneinrichtung (20, 20a) aufweist, und daß die Betätigungseinrichtungen in der Sammelbetriebsart betätigbar sind, um eine Blutmenge von der Zugangseinrichtung (10c) durch die dritte Durchflußbahn (46, 54) zu dem Reservoir (16, 16a) zu fördern, während gleichzeitig eine andere Blutmenge von der Zugangseinrichtung (10c) durch die erste Bahn (54) zu der Trenneinrichtung (20, 20a) gefördert wird.

2. Vorrichtung nach Anspruch 1, die eine Einrichtung (16, 16a) aufweist, um den ersten Bestandteil zur Rückleitung zu der Zugangseinrichtung (10c) zu sammeln.

3. Vorrichtung nach Anspruch 2, wobei die Sammeleinrichtung (16, 16a) für den ersten Bestandteil das Reservoir (16, 16a) zum Sammeln von Vollblut ist.

4. Vorrichtung nach Anspruch 2, wobei die Sammeleinrichtung (64) für den ersten Bestandteil von der Sammeleinrichtung (66) für Vollblut getrennt ist.

5. Vorrichtung nach Anspruch 1, wobei in der zweiten Betriebsart Vollblut zu der Trenneinrichtung (20, 20a) gefördert wird, das im wesentlichen frei von Vermischung mit dem ersten Bestandteil ist.

6. Vorrichtung nach Anspruch 1 mit einer Leitungseinrichtung zwischen der Trenneinrichtung (20, 20a) und dem Reservoir (16, 16a), um den ersten Bestandteil für die Rückleitung zum Spender von der Trenneinrichtung zu dem Reservoir zu transportieren.

7. Vorrichtung nach Anspruch 4, die eine Leitungseinrichtung zwischen der Trenneinrichtung (20, 20a) und der Sammeleinrichtung (64) für den ersten Bestandteil und eine Leitungseinrichtung (48) zwischen dieser Sammeleinrichtung (64) und der Zugangseinrichtung (10c) aufweist.

8. Vorrichtung nach Anspruch 4 oder 7, wobei die Sammeleinrichtung für Vollblut und den ersten Bestandteil eine erste und eine zweite Kammer (66, 64) aufweist, die in einem gemeinsamen Gehäuse (16a) ausgebildet sind.

9. Verfahren zum Trennen von Blut in Bestandteile unter Verwendung einer Trenneinrichtung (20, 20a), um das Blut in einen ersten und einen zweiten Bestandteil zu trennen, wobei das Verfahren aufweist: gesondertes Sammeln des ersten und des zweiten Bestandteils unter gleichzeitigem Fördern des Bluts zu einem Reservoir (16, 16a, 66) und von dem Reservoir, und anschließendes Rückleiten des ersten Bestandteils unter gleichzeitiger Durchführung des Trennschritts, wobei das Verfahren umfaßt: Zuführen von gesammeltem Blut aus dem Reservoir zu der Trenneinrichtung (20, 20a) zum Trennen, während gleichzeitig der erste Bestandteil rückgeleitet wird, so daß der Trennschritt kontinuierlich ist, DADURCH gekennzeichnet, daß während des Sammelns ein Teil des Bluts unter Umgehung des Reservoirs zu der Trenneinrichtung umgeleitet wird.

10. Verfahren nach Anspruch 9, wobei das Blut einem Zulauf (50) zugeführt wird, der allgemein an dem oberen Bereich des Reservoirs (66) liegt, wobei das Reservoir eine erste und eine zweite Kammer (66, 69) hat, und wobei der erste Bestandteil von der Trenneinrichtung (20, 20a) einem Zulauf zugeführt wird, der im allgemeinen an dem oberen Bereich der zweiten Kammer (69) des Reservoirs (16) liegt, wobei zwischen den Kammern (66, 69) Kommunikation besteht.

11. Verfahren nach Anspruch 10, wobei der Trenneinrichtung (20, 20a) während des Sammelzyklus Blut mit einer Rate zugeführt wird, die im wesentlichen gleich der Rate ist, mit der Vollblut zugeführt wird, abzüglich der Raten, mit denen der erste Blutbestandteil der ersten Kammer (66) zugeführt wird.

12. Verfahren nach Anspruch 10 oder 11, das während des Sammelzyklus die Schritte aufweist: Zuführen von Blut zu der ersten Kammer (66) mit einer Rate, die im wesentlichen gleich der Rate ist, mit der der erste Blutbestandteil der zweiten Kammer (69) zugeführt wird, so daß die Blutspiegel in den Kammern im wesentlichen auf gleichen Pegeln bleiben.

13. Verfahren nach einem der Ansprüche 10 bis 12, wobei die erste und die zweite Kammer (66, 69) gleiche Größe haben, um sowohl während Sammel- als auch während Rücklaufzyklen im wesentlichen die gleichen Pegel darin aufrechtzuerhalten.

14. Verfahren nach einem der Ansprüche 10 bis 13, das während des Rücklaufzyklus die Schritte aufweist: Zuführen von Blut aus der ersten Kammer (66) zu der Trenneinrichtung (20, 20a) mit einer Rate, die im wesentlichen gleich der Rate ist, mit der der erste Blutbestandteil aus der zweiten Kammer (69) rückgeleitet wird, abzüglich der Rate, mit der der erste Blutbestandteil aus der Trenneinrichtung (20, 20a) der zweiten Kammer (69) zugeführt wird.

15. Geräteset zur Verwendung in einer Vorrichtung zum Trennen des von einem Spender erhaltenen Bluts in Bestandteile, wobei das Geräteset folgendes aufweist: eine Trenneinrichtung (20, 20a) zum Trennen der Blutbestandteile; ein Blutreservoir (16, 16a); eine Venenpunktionsnadel (10c); eine erste Einrichtung (10, 10a, 46) zum Herstellen einer Blutflüssigkeitsverbindung zwischen der Nadel (10c) und dem Reservoir (16, 16a); eine zweite Einrichtung (10, 24; 10a, 54) zum Herstellen einer Blutflüssigkeitsverbindung zwischen der Nadel (10c) und der Trenneinrichtung (20, 20a); eine dritte Einrichtung (22, 42) zum Herstellen einer Blutflüssigkeitsverbindung zwischen der Trenneinrichtung (20, 20a) und dem Reservoir; und eine vierte Einrichtung (10, 10a, 48) zum Herstellen einer Blutflüssigkeitsverbindung zwischen dem Reservoir (16, 16a) und der Nadel (10c), wobei Blutflüssigkeit von dem Spender in dem Reservoir (16, 16a) gesammelt werden kann, während gleichzeitig Blutflüssigkeit von dem Spender der Trenneinrichtung (20, 20a) zugeführt wird, und wobei Blutflüssigkeit von dem Reservoir (16, 16a) der Trenneinrichtung (20, 20a) zugeführt werden kann, während gleichzeitig Blutflüssigkeit von dem Reservoir zu dem Spender rückgeleitet wird.

## Revendications

1. Appareil convenant pour séparer le sang provenant d'un donneur en ses constituants, qui comprend un moyen d'accès (10c) pour envoyer le sang d'un donneur a un séparateur (20, 20a) par un circuit d'écoulement (46, 54, 50, 48) afin de séparer le sang en un premier et un deuxième constituants, un réservoir (16, 16a) en communication dans ledit circuit d'écoulement avec le séparateur (20, 20a) par un premier trajet d'écoulement (50, 54) et avec le moyen d'accès (10c) par un deuxième trajet d'écoulement (46, 50), le circuit d'écoulement comprenant un trajet de retour (48) entre le séparateur (20, 20a) et le moyen d'accès (10c), un moyen d'actionnement (P1, P3, P4) ayant un mode de collecte et un mode de réinjection, le moyen d'actionnement pouvant fonctionner en mode de collecte pour envoyer du sang du moyen d'accès (10c) au réservoir (16, 16a) et au séparateur (20, 20a) et pouvant fonctionner en mode de réinjection pour envoyer le premier constituant séparé par le séparateur (20, 20a) vers le moyen d'accès (10c) par le trajet de retour (48), et un moyen pour fonctionner alternativement selon lesdits modes, le moyen d'actionnement (P1, P3, P4) pouvant fonctionner en mode de réinjection pour envoyer du sang provenant du réservoir (16, 16a) vers le séparateur (20, 20a) en même temps que ladite fourniture du premier constituant vers le moyen d'accès (10c) avec une alimentation continue de sang vers le séparateur (20, 20a), caractérisé en ce que le circuit d'écoulement comprend un troisième trajet d'écoulement (46, 54) évitant le réservoir entre le moyen d'accès (10c) et le séparateur (20, 20a), le moyen d'actionnement pouvant fonctionner en mode de collecte pour envoyer une quantité de sang provenant du moyen d'accès (10c) par le troisième trajet d'écoulement (46, 54) vers le réservoir (16, 16a) alors que, simultanément, une autre quantité de sang est envoyée du moyen d'accès (10c) vers le séparateur (20, 20a) par le premier trajet (54).

2. Appareil selon la revendication 1, comprenant un moyen (16, 16a) pour collecter le premier constituant pour le renvoyer au moyen d'accès (10c).

3. Appareil selon la revendication 2, dans lequel le moyen de collecte (16, 16a) pour le premier constituant est dans le réservoir (16, 16a) pour collecter le sang complet.

4. Appareil selon la revendication 2, dans lequel le moyen de collecte (64) pour le premier constituant est séparé du moyen de collecte (66) pour le sang complet.

5. Appareil selon la revendication 1, dans lequel, dans le deuxième mode de fonctionnement, le sang complet est envoyé au séparateur (20, 20a) en étant essentiellement exempt de mélange avec le premier constituant.

6. Appareil selon la revendication 1, comprenant un moyen de conduite entre le séparateur (20, 20a) et le réservoir (16, 16a) pour envoyer le premier constituant provenant du séparateur vers le réservoir pour être retourné au donneur.

7. Appareil selon la revendication 4, comprenant un moyen de conduite entre le séparateur (20, 20a) et le moyen de collecte (64) pour le premier constituant, et un moyen de conduite (48) entre ce moyen de collecte (64) et le moyen d'accès (10c).

8. Appareil selon la revendication 4 ou 7, dans lequel les moyens de collecte pour le sang complet et le premier constituant comprennent un premier et un deuxième compartiments (66, 64) disposés dans un logement commun (16a).

9. Procédé de séparation du sang en ses constituants en utilisant un séparateur (20, 20a) pour séparer le sang en un premier et un deuxième constituants, le procédé consistant à recueillir séparément les premier et deuxième constituants tout en envoyant le sang à un réservoir (16, 16a, 66) et, de ce réservoir, vers le séparateur et en renvoyant ensuite le premier constituant lorsqu'on effectue l'opération de séparation, le procédé comprenant l'envoi du sang recueilli provenant du réservoir vers le séparateur (20, 20a) pour être séparé, tandis que le premier constituant est renvoyé, si bien que l'étape de séparation est continue, caractérisé par la soustraction d'une partie du sang,pendant la collecte, envoyée au séparateur en évitant le réservoir.

10. Procédé selon la revendication 9, dans lequel le sang est envoyé à une entrée (50) située généralement dans la partie supérieure du réservoir (66), le réservoir ayant des premier et deuxième compartiments (66, 69) et dans lequel le premier constituant est envoyé du séparateur (20, 20a) vers une entrée située généralement dans la partie supérieure du deuxième compartiment (69) du réservoir (16), une communication étant assurée entre les compartiments (66, 69).

11. Procédé selon la revendication 10, comprenant, pendant le cycle de collecte, l'envoi du sang vers le séparateur (20, 20a) par un débit essentiellement égal au débit de fourniture du sang complet moins les débits selon lesquels le premier constituant du sang est envoyé au premier compartiment (66).

12. Procédé selon la revendication 10 ou 11 comprenant, pendant le cycle de collecte, les opérations consistant à envoyer le sang vers le premier compartiment (66) à un débit essentiellement égal au débit de fourniture du premier constituant du sang au deuxième compartiment (69), alors que les niveaux de sang dans lesdits compartiments restent à des niveaux essentiellement égaux.

13. Procédé selon l'une quelconque des revendications 10 à 12, dans lequel les premier et deuxième compartiments (66, 69) ont une dimension telle que des niveaux essentiellemnt égaux sont maintenus dans ces compartiments pendant les deux cycles de collecte et de retour.

14. Procédé selon l'une quelconque des revendications 10 à 13 comprenant, pendant le cycle de retour, les opérations consistant à envoyer le sang provenant dudit premier compartiment (66) vers ledit séparateur (20, 20a), à un débit essentiellement égal au débit de retour du premier constituant du sang provenant dudit deuxième compartiment (69), moins le débit d'alimentation du premier constituant du sang vers ledit deuxième compartiment (69) en provenance dudit séparateur (20, 20a).

15. Dispositif à utiliser dans un appareil pour séparer le sang provenant d'un donneur en ses constituants, le dispositif comprenant un séparateur (20, 20a) pour séparer les constituants du sang, un réservoir à sang (16, 16a); une aiguille pour poinçonner la veine (10c); un premier moyen (10, 10a, 46) pour établir une communication pour le sang entre ladite aiguille (10c) et le réservoir (16, 16a); un deuxième moyen (10, 24:10a, 54) pour établir une communication pour le sang entre l'aiguille (10c) et le séparateur (20, 20a); un troisième moyen (22, 42) pour établir une communication pour le sang entre le séparateur (20, 20a) et le réservoir; et un quatrième moyen (10, 10a, 48) pour établir une communication pour le sang entre le réservoir (16, 16a) et l'aiguille (10c) alors que le sang provenant du donneur peut être collecté dans le réservoir (16, 16a) tandis que le sang est envoyé simultanément du donneur vers le séparateur (20, 20a) et que le sang provenant du réservoir (16, 16a) peut être envoyé au donneur séparateur pendant que du sang est renvoyé du réservoir au donneur.
